# EUROPÄISCHE PATENTSCHRIFT

(11) **EP 1 058 542 B1**
(45) Veröffentlichungstag und Bekanntmachung des Hinweises auf die Patenterteilung: **31.08.2005**
(21) Anmeldenummer: 99908932.9
(22) Anmeldetag: 24.02.1999
(51) Int. Cl.: A61K 9/16, A61K 47/40, A61K 31/557

(54) **VERFAHREN ZUR HERSTELLUNG VON UMMANTELTEN, SPHÄRISCHEN GRANULATKÖRNERN ENTHALTEND PROSTAN-DERIVATE**
METHOD FOR PRODUCING ENCASED SPHERICAL GRANULAR GRAINS COMPRISING PROSTANE DERIVATIVES
PROCEDE DE PREPARATION DE GRAINS GRANULAIRES SPHERIQUES ENROBES CONTENANT DES DERIVEES DU PROSTAN

(30) Priorität: 24.02.1998 DE 19808634
(43) Veröffentlichungstag der Anmeldung: 13.12.2000
(73) Patentinhaber: Schering Aktiengesellschaft, 13342 Berlin (DE)
(72) Erfinder: WAGNER, Torsten, D-13127 Berlin (DE)
(86) Internationale Anmeldenummer: PCT/EP1999/001100
(87) Internationale Veröffentlichungsnummer: WO 1999/043303

(56) Entgegenhaltungen:
- WO-A-87/05294
- WO-A-98/10751
- DE-A- 3 622 487
- GB-A- 1 251 115
- SHUN POR LI ET AL.: "Preparation and in vitro evaluation of a controlled release drug delivery system of Theophylline using an aqueous acrylic resin dispersion" DRUG DEVELOPMENTS AND INDUSTRIAL PHARMACY, Bd. 15, Nr. 8, 1989, Seiten 1231-1242, XP002106507 in der Anmeldung erwähnt

## Beschreibung

Die Erfindung betrifft ein Verfahren zur Herstellung von ummantelten, sphärischen Granulatkörnern enthaltend Prostan-Derivate.

### Stand der Technik

Prostan - Derivate und deren Herstellung werden in der EP 0 011 591 (Anmeldetag: 18.10.1979) ausführlich beschrieben. Bei den Prostan - Derivaten handelt es sich um Verbindungen, die vom Prostacyclin (PGI₂) abgeleitet sind. Sie enthalten eine Mehtylengruppe an Stelle des 9 - Ether - Sauerstoffatoms im Prostacyclin. Prostan - Derivate werden zur Behandlung von verschiedenen Krankheiten eingesetzt, wobei die cardiovaskuläre und thromboaggregationshemmende Wirkung klar im Vordergrund steht. Die Verwendung von Prostan - Derivaten als Medikament wird ausführlich in der europäischen Publikation EP 0 011 591 beschrieben. In den Publikationen EP 0 055 208, EP 0 099 538 und EP 0 119 949 werden Carbacyclin - Derivate aufgeführt, die ähnliche Indikationen wie die zuvor erwähnten Prostan - Derivate besitzen. Weitere Prostan - Derivate sind in der Publikation EP 0 084 856 beschrieben, die für die Verwendung bei der Inhibierung der Thrombozyten - Aggregation, Senkung des systemischen Blutdrucks oder der Behandlung von Magengeschwüren vorgeschlagen wurden. Iloprost ist das wichtigste Prostan - Derivat.

Bei Iloprost oral (pharmazeutischer Wirkstoff: 5-(E)-(1S,5S,6R)-7-Hydroxy-6[(E)-(3S,4RS)-3-hydroxy-4-methyl-1-octen-6-inyl]bicyclo[3.3.0]octen-3-yliden pentansäure) handelt es sich um eine Retardkapsel. Retardkapseln zählen zu den multipartikulären Arzneiformen (multiple units). Sie bestehen aus einer Hartgelatinekapsel, die mit vielen kleinen Retardkörpern (bei Iloprost oral: überzogene Pellets [ = sphärische Granulatkömer]) gefüllt ist. Nach peroraler Applikation löst sich die Hartgelatinekapsel im Magen rasch auf und die einzelnen Retardkörper werden freigesetzt. Von dort aus gelangen sie unabhängig vom Füllungszustand des Magens weiter in den Darm. Gleichzeitig wird der enthaltene Wirkstoff kontinuierlich abgegeben. Diffusionspellets stellen die wichtigste Gruppe der überzogenen multipartikulären Arzneiformen dar.

Als Überzugsmaterialien für perorale Retardarzneiformen haben Polymere eine herausragende Bedeutung erlangt. Bei den verwendeten Polymertypen handelt es sich in erster Linie um Ethylcellulose oder Poly(meth)acrylsäureester. Die Polymere können entweder als organische Lösung oder in wäßriger Phase dispergiert angewendet werden. Da sie in der Handhabung umweltfreundlicher, sicherer und kostengünstiger sind, werden in der Industrie zunehmend wäßrige Polymerdispersionen eingesetzt. Die bekanntesten Handelsnamen sind Aquacoat ECD 30®, Surelease® oder Eudragit® NE 30 D, RL 30 D und RS 30 D. In den für den Coatingprozeß verwendeten Lacksuspensionen sind zusätzliche Additive enthalten, die herstellungs- und anwendungsbedingt erforderlich sind und die Eigenschaften des Filmbildners mitbestimmen.

Verfahrenstechnisch erfolgt der Umhüllungsprozeß von Pellets mit einer Polymermembran in Wirbelschicht-Geräten. Durch einen Luftstrom ordnen sich die Pellets zu einem Wirbel- oder Fließbett an, auf das je nach Gerätetyp im Gegen- oder Gteichstrom (Top- bzw. Bottom-Spraying) aus einer Sprüheinrichtung (eine oder mehrere Sprühdüsen) die Polymerdispersion mit den zugesetzten Additiven gesprüht wird. Durch die Bewegung in der Wirbelschicht verteilt sich die feinteilige Dispersion auf der Oberfläche der Pellets. Gleichzeitig verdunstet das Dispersionsmittel Wasser durch den Trocknungsstrom der Luft, und die Latexpartikel nähern sich zu einer immer dichter werdenden Kugelpackung an. Anschließend findet eine zunehmende Durchdringung der Partikel untereinander statt. Dieser Vorgang wird als Koaleszenz bezeichnet. Im Endstadium dieses Prozesses entsteht ein fast homogener, in Wasser unlöslicher Film. Der das Pellet jetzt umgebene Film wirkt als Diffusionsbarriere für den im Kern befindlichen Arzneistoff und führt zu einer verzögerten Arzneistofffreisetzung.

Die Herstellung von Iloprost oral wird in zwei wesentlichen Schritten durchgeführt. Im ersten Schritt werden wirkstoffhaltige Rohpellets über einen Extrusions-/Sphäronisationsprozeß hergestellt. Die Rohpellets bestehen zu 90 % aus Lactose sowie 10 % Avicel PH 101 als Hilfsstoffe und enthalten als Wirkstoff Iloprost-β-Cyclodextrin Clathrat. Der Gehalt beträgt 0,05 bzw. 0,1 mg Iloprost pro 65 bzw. 130 mg Pellets [Wirkstoffgehalt ca. 0,08 % (w/w)] In einem zweiten Schritt werden die Rohpellets dann mit einer Coatingsuspension, basierend auf Eudragit NE 30 D und Additiven in einem Wirbelschichtgerät gecoatet.

Zu Beginn der Entwicklung wurden die Rohpellets in einem Wirbelschichtgerät (WSG), Typ GPCG-3 (Fa. Glatt)) bzw. Typ Strea (Fa. Aeromatic) im Labormaßstab (beides Top-Spraying-Verfahren) im Prinzip mit der qualitativ gleichen Coatingsuspension besprüht, die heute auch noch verwendet wird. Als Zielgröße für den Lackauftrag diente das resultierende Wirkstofffreisetzungsprofil (Bestimmung der freigesetzten Wirkstoffmenge nach z.B. 1, 2 und 3 Stunden), das im Dissolutionstest nach USP, Apparat I (basket) untersucht wurde. Die hergestellten Pellets wurden für erste kinetische und klinische Studien verwendet. Ein Problem von Anfang an war die Tatsache, daß bei dieser Formulierung die Wirkstofffreisetzung sich über die Lagerzeit etwas verlangsamte. Die ersten größeren Chargen (Produktionsmaßstab) wurden im WSG Aeromatic MP4 (Fa. Aeromatic) gecoatet. Genauso wie in den kleineren Laborgeräten fand der Sprühauftrag im Gegenstromverfahren (Top-Spraying) statt. Dabei wurde aus einer Sprühdüse von oben in entgegengesetzter Richtung zum Zuluftstrom auf die in der Wirbelschicht befindlichen Pellets gesprüht. Im Aeromatic MP4 wurden im Endeffekt jeweils 75 kg wirkstoffhaltige Rohpellets mit 16,5 kg Lacksuspension besprüht.

Bei der Verfahrensübertragung vom Labormaßstab auf den Aeromatic MP4 fiel dann auf, daß die Wirkstoffreisetzung sich über die Lagerzeit verlangsamte und aus diesem Grunde die zuerst mit höheren Lackmengen als 16,5 kg hergestellten Chargen schon bald nicht mehr der Spezifikation entsprachen. Dieses für diese Formulierung festgestellte Phänomen wird in der Posterveröffentlichung von T.C. WAGNER und S. KEITEL, The effect of dispersion concentration and curing temperature on drug release of pellets coated with Eudragit NE 30 D, Proc. 1st World Meeting APGI/APV, Budapest, 9-11 May 1995 beschrieben. Hierzu wurde erstmals der Einfluß einer thermischen Nachbehandlung (Temperung) auf die Wirkstofffreisetzung untersucht. Für den Produktionsmaßstab wurde jedoch generell auf eine Temperung verzichtet, da keine Trockenschränke zur Verfügung standen, das genaue Ausmaß dieses Problems noch nicht bekannt war und weiterhin aufgrund von thermischen Instabilitäten des Wirkstoffs zunächst grundsätzliche Bedenken bestanden . Aus diesem Grunde mußte für die Herstellung eine Lackmenge aufgetragen werden, die nach der Herstellung zu einem Wirkstofffreisetzungsprofil außerhalb der Spezifikation führte. Nach ca. 4 Wochen Lagerzeit bei Raumtemperatur hatte sich die Freisetzung verlangsamt und das WSF-Profil lag im oberen Drittel der Spezifikation. Über die Lagerzeit jedoch trat eine weitere Verlangsamung ein.

In der Publikation F.W. GOODHART et al. (1984) An evaluation of aqueous filmforming dispersions for controlled release, in Pharmaceutical Technology, Vol. p 65 - 71 werden Verfahren beschrieben, bei denen das Tempern zu wissenschaftlichen Zwecken geschieht. Es ist Sinn und Zweck, hinter die Phänomene zu kommen. Der Effekt, die Stabilität zu steigern, wird nicht erwähnt.

In der Publikation Shun Por LI et al. (1989) Preparation and *In - Vitro* evaluation of a controlled release drug delivery system of Theophylline using an aqueous acrylic resin dispersion, in Drug Development and Industrial Pharmacy, Vol. 15(8), pp 1231 - 1242 wird ein Tempern beschrieben, bei dem der Wirkstoff Theophillin mit Poly(meth)acrylsäureester ummantelt wird. Das Tempern wird bei 40 °C über 24 Stunden ausgeführt. Durch das Tempern wird bei diesem Wirkstoff die Stabilität gesteigert.

### Aufgabe und Lösung

Es stellt sich die Aufgabe, ein Verfahren zur Herstellung eines Mantels für sphärische Granulatkörner enthaltend Prostan-Derivate anzubieten, mit dem ein Wirkstofffreisetzungsprofil erhalten werden kann, welches auch nach längerer Lagerzeit innerhalb der Spezifikation liegt.

Die Aufgabe wird gelöst durch ein Verfahren zur Herstellung von ummantelten, sphärischen Granulatkörner (Pellets), wobei das Verfahren die folgenden Schritte umfaßt:
α) in einem Wirbelbett oder Fließbett werden wirkstoffhaltige Rohpellets aus mindestens einem pharmazeutischem Wirkstoff und gegebenenfalls pharmazeutisch verträglichen Zusatzstoffen und / oder Trägern mit einer Polymerdisperson und Additiven besprüht, die eine Ethylcellulose und / oder ein Poly(meth)acrylsäureester als Polymer umfaßt,
β) eine Polymerdispersion wird mit einer Polymerdicke im Bereich von 1 bis 5 % (w/w) der Gesamtmasse aufgetragen,
   wobei die Gesamtmasse der Zusammensetzung der ummantelten Pellets entspricht
γ) die mit Polymer ummantelten. Pellets werden bei einer Temperatur von 45 bis 65 °C getempert,
δ) die Temperung der mit dem Polymer ummantelten Pellets dauert mindestens 24 Stunden, und
ε) der pharmazeutische Wirkstoff ist ein Clathrat eines Prostan - Derivates ist.

Bevorzugt ist ein Wirbelbett oder Fließbett, bei dem die Pellets in einem Gleichstromverfahren besprüht werden. Mehr bevorzugt ist ein Gleichstromverfahren, bei dem aus mindestens 1 oder 2, bevorzugt 12 Düsen die Polymerdispersion (und zugesetzte Additive) in das Wirbelbett oder Fließbett gesprüht wird.

Bevorzugt ist, daß die Polymerdisperson entweder Ethylcellulose oder ein Poly(meth)acrylsäureester als Polymer umfaßt.

Vorteilhaft ist, wenn die Dicke des aufgetragenen Polymers gleichmäßig über das Pellet verteilt ist. So sollten die Toleranzen der Polymerdicke vorteilhafterweise nur ± 30%, bevorzugt ± 20% und mehr bevorzugt ± 10%sein.

Bevorzugt ist ein erfindungsgemäßes Verfahren, bei dem die ummantelten Pellets anschließend in eine Tabletten oder eine Retardkapsel überführt werden, die beispielsweise Hartgelatinekapseln sind. Die Abfüllung in Kapseln ist bevorzugt. Auch andere Abfüllungen oder Behältnisse von ummantelten Pellets sind möglich. Solche Behältnisse können in IUDs vorliegen, die dort ein Steroid retardiert freisetzen. Ebenso können die Abfüllungen als Tabletten mit leicht lösbarer Preßmasse eingesetzt werden. Weiterhin sind Behältnisse als Implantate möglich.

Bevorzugt ist ein erfindungsgemäßes Verfahren, wobei die Pellets als Wirkstoff Prostan-Derivate und pharmazeutische Träger und / oder Zusatzstoff umfassen. Am meisten bevorzugt ist der Wirkstoff 5-(E)-(1 S,5S,6R)-7-Hydroxy-6[(E)-(3S,4RS)-3-hydroxy-4-methyl-1-octen-6-inyl]bicyclo[3.3.0]octen-3-yliden pentan-säure und als Zusatzstoff mindestens Cyclodextrin

### Vorteile

In Optimierungsphasen wurden die erfindungsgemäßen Temperungsbedingungen entwickelt. Die Temperung bei 50 °C über 48 h ist geeignet, um bei der jetzt vorliegenden Formulierung die Wirkstofffreisetzung reproduzierbar zu stabilisieren und über die Lagerzeit konstant zu halten.

Der Unterschied des erfindungsgemäßen Verfahren gegenüber der Publikation von Shun Por LI et al. (1989) Drug Development and Industrial Pharmacy, Vol. 15(8), pp 1231 - 1242 besteht darin, daß Prostan - Derivate im Gegensatz zu Theophillin nicht stabilisiert werden, wenn sie bei 40 °C über 24 Stunden getempert werden. Daher beschreibt diese Publikation einen Weg, der ein stubstanzspezifisches Tempern zur Folge hat. Die in dem Dokument erwähnte Technik ist nicht auf Prostan - Derivate übertragbar und führt bei den Prostan - Derivaten nicht zu der gewünschten Wirkung.

### Bevorzugte Ausführungsformen

Ethylcellulose ist beschrieben in Römpp Chemie Lexikon, 9. Auflage, 1990, Herausg. J. FALBE und M. REGITZ, Georg Thieme Verlag, Stuttgart (ISBN 3-13-734709-2) auf der Seite 1254.

Bevorzugt ist als Polymer der Poly(meth)acrylsäureester, welcher in dem Europäischen Arzneibuch, 3. Ausgabe, 1997, Deutscher Apotheker Verlag Stuttgart, Eschborn, auf der Seite 1516 bis Seite 1517 unter Punkt 733 beschrieben ist.

Am meisten bevorzugt ist als Polymer EUDRAGIT NE 30 D, welches in den folgenden Publikationen beschrieben ist:
- K. LEHMANNN and D. DREHER (1979) Coating small particles with acylic resins, Pharmaceutical Technology, Vol. 3 (3);
- K. LEHMANN (1984) Formulation of controlled release tablets with acrylic resins, Acta Pharm. Fenn. Vol. 93, pp 55;
- K. LEHMANN, (1986) Acrylic latices from redispersable powders for peroral and transdermal drug formulations, Drug Development and Industrial Pharmacy, Vol. 12(3), pp 265;
- K. LEHMANN and D. DREHER (1986) Mischbarkeit wäßriger Poly(meth)acrylat-Dispersionen für Arzneimittelüberzüge, Pharm. Ind. Vol 48, pp 1182;
- K. LEHMANN and H:U: PETEREIT (1988) Verwendung wäßriger Poly(Meth)acrylat-Dispersionen für die Herstellung von Matrixtabletten, (1988) Acta Pharma. Tech. Vol. 34(4), pp 189 und
- K. LEHMANN et al. (1989) Praktikum des Lackdragierens.

Die Pellets umfassen als Rohpellets den pharmazeutischen Wirkstoff und weiterhin auch pharmazeutisch verträgliche Träger und Zusatzstoffe. Solche Träger und Zusatzstoffe sind in Remington's Pharmaceutical Science, 15^{th} ed. Mack Publishing Company, Easton Pennsylvania (1980) beschrieben.

Mehr bevorzugt ist als Träger Cyclodextrin. Cyclodextrin wird nach dem Abbau von Stärke durch Bacillus macerans oder Bacillus circulans unter Einwirkung von Cyclodextringlycosyltransferase gebildet. Die Cyclodextrine besteh aus 6, 7 oder 8 α-1-4-verknüpfte Glucoseeinheiten (α-, β- und γ-Cylclodextrine). Die Cyclohexa- (hepta-, octa-)amylosen sind im Kristallgitter der Cyclodextrine so aufeinandergeschichtet, daß sie durchgehende innermolekulare Kanäle bilden, in denen sie hydrophobe Moleküle in wechselnden Mengen einschließen können. (Literatur: Adv. Carbohydr. Chem. Vol. 12, p 189 (1987), Angew. Chem. Vol. 92, p 343 (1980), Bender u. Komiyama, Cyclodextrin Chemistry, Berlin: Springer 1978, Naturwissenschaften Vol. 154, p 625 (1967))

Der pharmazeutischer Wirkstoff betrifft Prostan-Derivate der allgemeinen Formel I oder II 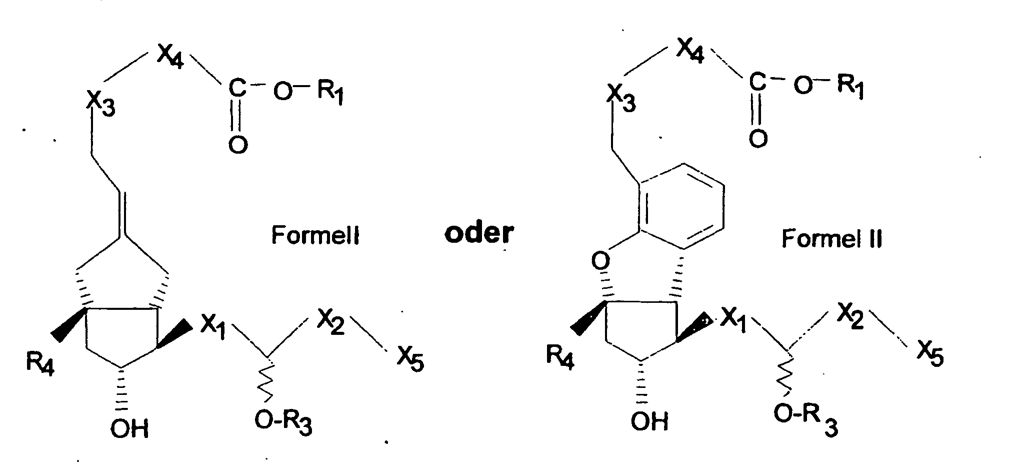 worin
- X₁: ein -CH₂-CH₂-; trans -CH=CH- oder -C≡C- ist,

- X₂: eine geradkettige oder verzweigte gesättigte Alkylengruppe mit 1 bis 6 Kohlenstoff-Atomen ist,
- X₃: ein -O- oder -CH₂- ist,
- X₄: ein -CH₂- oder -[CH₂]₃- ist,
- X₅: ein -H oder -C≡C-R₂ ist;
- R₁: ein Wasserstoff-Atom, eine Alkylgruppe mit 1 bis 6 Kohlenstoff-Atomen, eine Cycloalkylgruppe mit 5 oder 6 Kohlenstoff-Atomen oder Phenylgruppe ist,
- R₂: eine gerad- oder verzweigtkettige, gesättigte oder ungesättigte Alkylgruppe mit 1 bis 6 Kohlenstoff-Atomen ist,
- R₃: ein Wasserstoff-Atom, ein Acylrest mit 1 bis 4 Kohlenstoff- Atomen oder ein Benzoylrest ist und
- R₄: ein -H oder -CH₃ ist;
wobei die -O-R₃-Gruppe a- oder b- ständig ist,
und deren Salze mit physiologisch verträglichen Basen, falls R₁ die Bedeutung eines Wasserstoff-Atoms hat.

Die Publikationen EP 0 011 591, EP 0 055 208, EP 0 099 538, EP 0 119 949, EP 0 084 856 und EP 0 686 036 beschreiben die Prostan - Derivate, deren Herstellung und Verwendung.

Zur Salzbildung mit den freien Säuren sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin u.s.w. Die β-Cyclodextrinclathrate werden entsprechend EP 0 259 468 hergestellt.

Bevorzugt ist die Verwendung von erfindungsgemäßen Prostan-Derivaten mit der zuvor genannten allgemeinen Formel I
worin
- X₁: ein trans -CH=CH- ist,
- X₂: eine geradkettige oder verzweigte gesättigte Alkylengruppe mit 2 bis 4 Kohlenstoff-Atomen ist,
- X₃: ein -CH₂- ist,
- X₄: ein -CH₂- ist,

- X₅: ein -C≡C-R₂ ist;
- R₁: ein Wasserstoff-Atom, eine Alkylgruppe mit 1 bis 3 Kohlenstoff-Atomen oder Phenylgruppe ist,
- R₂: eine gerad- oder verzweigtkettige, gesättigte oder ungesättigte Alkylgruppe mit 1 bis 3 Kohlenstoff-Atomen ist,
- R₃: ein Wasserstoff-Atom oder ein Acylrest mit 2 Kohlenstoff Atomen ist und
- R₄: ein -H ist;
wobei die -O-R₃-Gruppe a- oder b- ständig ist,
und deren Salze mit physiologisch verträglichen Basen, falls R₁ die Bedeutung eines Wasserstoff-Atoms hat.

Am meisten bevorzugt ist die Verwendung von einem Prostan-Derivat mit dem Namen "Iloprost" und welches die systematische Bezeichnung 5-(E)-(1 S,5S,6R)-7-Hydroxy-6[(E)-(3S,4RS)-3-hydroxy-4-methyl-1-octen-6-inyl]bicyclo[3.3.0]octen-3-yliden pentansäure trägt.

Weiterhin umfaßt als Wirkstoff auch die Substanzen Cicaprost, Eptaloprost, Ciprosten und/oder Beraprost und deren Salze.

Pharmazeutische Wirkstoffe können auch als niedrig dosierte Wirkstoffe vorliegen, hierbei handelt es sich um Wirkstoffgehalte von 1 % und weniger (w/w) bezogen auf die Gesamtmasse aus Wirkstoff und Träger und Zusatzstoff. Bevorzugt ist ein Prozentsatz von 0,3 und weniger; mehr bevorzugt von 0,1 und weniger.

Bevorzugt ist eine Dicke des Polymers von 1,5 bis 4 % (w/w) der Gesamtmasse, mehr bevorzugt eine Dicke von 1,75 bis 3 % und am meisten bevorzugt eine Dicke von 2,1 % ± 10% (2,0 - 2,2 %).

Bevorzugt ist beim Tempem für den Temeperaturbereich die untere Temperaturgrenze 45 °C, mehr bevorzugt 47 °C, noch mehr bevorzugt 48 °C und am meisten bevorzugt 50 °C.
Bevorzugt ist beim Tempern für den Temeperaturbereich die obere Temperaturgrenze 65 °C, mehr bevorzugt 60 °C, noch mehr bevorzugt 47 °C und am meisten bevorzugt 53 °C.

Die Verweildauer beim Tempern beträgt mindestens 24 Stunden. Vergleichbar gute Ergebnisse werden durch ein längeres Tempern erzielt. Schlechtere Daten für das Wirkstoffreisetzungsprofil lassen sich durch ein Verkürzen erreichen. Üblicher Weise kann eine hohe Temperatur die Stabilität des Produktes beeinträchtigen. Daher sind möglichst kurze Zeiten und schonende Bedingungen beim Tempern wünschenswert. Am meisten bevorzugt ist eine Verweildauer von 48 h ± 5h.

### Beispiele:

### Beispiel 1 (altes Verfahren, Top-Spraying, Produktionsmaßstab, keine Temperung)

a) Wirkstoffhattige Rohpellets werden nach einem Extrusions/Spheronisationsverfahren hergestellt. Dazu wird eine wirkstoffhaltige Hilfsstoff / Wirkstoff - Vormischung (90 % Lactose, 90 % < 0,1 mm, 10 % Avicel PH 101 und Iloprost-β-Cyclodextrin Clathrat) in einem Mixer mit Wasser granuliert. In einem zweiten Schritt wird die feuchte Pulvermischung durch die Lochscheibe eines Extruders zu einem strangförmigen Granulat geformt, das in der Spheroniserzelle zu sphärischen Granulatkörnern (Pellets) verrundet wird. Anschließend werden die Rohpellets in einem Wirbelschichttrockner getrocknet.
b) 75 kg wirkstoffhaltige Rohpellets [Wirkstoffgehalt Iloprost 0,08 % (w/w)] werden im Top-Spaying-Verfahren in einem Aeromatic MP 4 - Coater mit 16,5 kg Coatingsuspension folgender Zusammensetzung besprüht:

| | |
|---|---|
| 7,063 kg | Eudragit® NE 30 D |
| | |
| 0,090 kg | Magnesiumstearat |
| 0,066 kg | Titandioxid |
| 0,040 kg | Polyethylenglykol 6000 |
| 0,046 kg | Polysorbat 80 |
| 9,195 kg | Wasser |
| 16,500 kg | Coatingsuspension |

Für den Sprühprozeß werden die folgenden Parameter eingestellt:

| | |
|---|---|
| Zulufttemperatur | 35 °C |
| Sprührate | 235 g/min |
| Sprühdüse | eine Zweistoffdüse, Durchmesser 1,8 mm |
| Sprühluftdruck | 4 bar |

Die so erhaltenen Retardpellets weisen die folgende Zusammensetzung auf:

| | |
|---|---|
| 0,050 mg | Iloprost |
| 0,330 mg | β-Cyclodextrin |
| 56,260 mg | Lactose, 90 % < 0,1 mm |
| 6,250 mg | Avicel PH 101 |
| 1,777 mg | Eudragit® NE |
| 0,075 mg | Magnesiumstearat |
| 0,055 mg | Titandioxid |
| 0,034 mg | Polyethylenglykol 6000 |
| 0,039 mg | Polysorbat 80 |
| 0,134 mg | Siliciumdioxid |
| 65,00 mg | Retardpellets |

Die in vitro Freigabeuntersuchung einer abgeteilten Dosis im Dissolutiontest, Apparat 1, basket, USP in Phosphatpufferlösung pH 7,4 führte zu folgenden Werten:

| Zeitpunkt der Untersuchung | Freigabe nach 1 Stunde | Freigabe nach 2 Stunden | Freigabe nach 3 Stunden |
|---|---|---|---|
| Start | 64,3 % | 86,8 % | 95,8 % |
| nach 4 Wochen Lagerung | 48,7 % | 75,7 % | 86,5 % |
| nach 3 Monaten Lagerung | 42,4 % | 70,9 % | 85,9 % |
| nach 24 Monaten Lagerung | 32,4 % | 58,3 % | 75,9 % |

Die Lagerung erfolgte bei Raumtemperatur. Die Abnahme der Freigaberaten über die Lagerzeit belegt, daß es sich nicht um ein stabiles Produkt handelt.

### Beispiel 2 (altes Verfahren, Top-Spraying, Labormaßstab, ungetempert und getempert bei 50 °C / 4 Stunden)

a) Wirkstoffhaltige Rohpellets werden wie in Beispiel 1a) hergestellt.
b) 750 g wirkstoffhaltige Rohpellets [Wirkstoffgehalt Iloprost 0,08 % (w/w)] werden im Top-Spaying-Verfahren in einem Aeromatic Strea1 - Coater mit 240 g Coatingsuspension folgender Zusammensetzung besprüht:

| | |
|---|---|
| 178,5 g | Eudragit® NE 30 D |
| 2,3 g | Magnesiumstearat |
| 1,7 g | Titandioxid |
| 1,0 | Polyethylenglykol 6000 |
| 1,2 g | Polysorbat 80 |
| 232,2 g | Wasser |
| 416,9 g | Coatingsuspension |

Für den Sprühprozeß werden die folgenden Parameter eingesteltt:

| | |
|---|---|
| Zutufttemperatur: | 28-32 °C |
| Sprührate | 4 g/min |
| Sprühdüse | eine Zweistoffdüse, Durchmesser 1,0 mm |
| Sprühluftdruck | 1,0 bar |

Nach dem Coatingprozeß wird ein Teil der Retardpellets bei 50 °C im Umluftrockenschrank für 4 Stunden getempert.

Die so erhaltenen Retardpellets weisen die folgende Zusammensetzung auf:

| | |
|---|---|
| 0,050 mg | Iloprost |
| 0,330 mg | β-Cyclodextrin |
| 54,632 mg | Lactose, 90 % < 0,1 mm |
| 6,113 mg | Avicel PH 101 |
| 2,549 mg | Eudragit® NE |
| 0,110 mg | Magnesiumstearat |
| 0,979 mg | Titandioxid |
| 0,048 mg | Polyethylenglykol 6000 |
| 0,057 mg | Polysorbat 80 |
| 0,132 mg | Siliciumdioxid |
| 65,000 mg | Retardpellets |

Die in vitro Freigabeuntersuchung einer abgeteilten Dosis im Dissolutiontest, Apparat 1, basket, USP in Phosphatpufferlösung pH 7,4 führte zu folgenden Werten:

| Zeitpunkt der Untersuchung | Freigabe nach 1 Stunde | Freigabe nach 2 Stunden | Freigabe nach 3 Stunden |
|---|---|---|---|
| Start (ungetempert) | 79,8 % | 97,3 % | 101,1 % |
| nach 4 Wochen Lagerung (ungetempert) | 63,4 % | 88,2 % | 100,4 % |
| Start (getempert: 50 °C / 4 Stunden) | 58,7 % | 85,9 % | 96,7 % |
| nach 4 Wochen Lagerung (getempert: 50 °C / 4 Stunden) | 49,8 % | 79,2 % | 94,3 % |

Die Lagerung erfolgte bei Raumtemperatur. Sowohl das ungetemperte als auch das bei 50 °C über 4 h getemperte Produkt erwiesen sich über eine Lagerzeit von 4 Wochen aufgrund der Abnahmen der Freigaberaten als nicht stabil.

### Beispiel 3 (modifiziertes, neues Verfahren, Bottom-Spraying, Produktionsmaßstab, Temperung bei 50 °C / 4 Stunden)

a) Wirkstoffhaltige Rohpellets werden wie in Beispiel 1a) hergestellt.
b) 45 kg wirkstoffhaltige Rohpellets [Wirkstoffgehalt Iloprost 0,08 % (w/w)] werden im Bottom-Spaying-Verfahren in einem Hüttlin HKC 50 Coater mit 7,7 kg Coatingsuspension folgender Zusammensetzung besprüht

| | |
|---|---|
| 3,297 kg | Eudragit®NE 30 D |
| 0,042 kg | Magnesiumstearat |
| 0,031 kg | Titandioxid |
| 0,019 kg | Polyethylenglykol 6000 |
| 0,021 kg | Polysorbat 80 |
| 4,290 kg | Wasser |
| 7,700 kg | Coatingsuspension |

Für den Sprühprozeß werden die folgenden Parameter eingestellt:

| | |
|---|---|
| Zulufttemperatur | 32 °C |
| Sprührate | 200 g/min |
| Sprühdüse | 12 Dreistoffdüsen, Durchmesser 0,8 mm |
| Sprühluftdruck | 1,5 bar |

Nach dem Coatingprozeß wird ein Teil der Retardpellets bei 50 °C im Umluftrockenschrank für 4 Stunden getempert.

Die so erhaltenen Retardpellets weisen die folgende Zusammensetzung auf:

| | |
|---|---|
| 0,050 mg | Iloprost |
| 0,330 mg | β-Cyclodextrin |
| 56,606 mg | Lactose, 90 % < 0,1 mm |
| 6,332 mg | Avicel PH 101 |
| 1,392 mg | Eudragit® NE |
| 0,059 mg | Magnesiumstearat |
| 0,044 mg | Titandioxid |
| 0,027 mg | Polyethylenglykol 6000 |
| 0,030 mg | Polysorbat 80 |
| 0,130 mg | Siliciumdioxid |
| 65,000 mg | Retardpellets |

Die in vitro Freigabeuntersuchung einer abgeteilten Dosis im Dissolutiontest, Apparat 1, basket, USP in Phosphatpufferlösung pH 7,4 führte zu folgenden Werten:

| Zeitpunkt der Untersuchung | Freigabe nach 1 Stunde | Freigabe nach 2 Stunden |
|---|---|---|
| Start (getempert: 50 °C / 4 Stunden) | 51,2% | 78,1 % |
| nach 4 Wochen Lagerung (getempert: 50 °C / 4 Stunden) | 46,3 % | 75,4 % |

Die Lagerung erfolgte bei Raumtemperatur. Wie in Beispiel 3 zeigt das bei 50 °C über 4 h getemperte Produkt eine Abnahme in den Freigaberaten über eine Lagerzeit von 4 Wochen. Das Produkt ist ebenfalls nicht stabil.

### Beispiel 4 (neues Verfahren / final process, Bottom-Spraying, Produktionsmaßstab, ungetempert und Temperung bei 50 °C / 48 Stunden)

a) Wirkstoffhaltige Rohpellets werden wie in Beispiel 1a) hergestellt.
b) 45 kg wirkstoffhaltige Rohpellets [Wirkstoffgehalt Iloprost 0,08 % (w/w)] werden im Bottom-Spaying-Verfahren in einem Hüttlin HKC 50 Coater mit 7,6 kg Coatingsuspension folgender Zusammensetzung besprüht:

| | |
|---|---|
| 3,253 kg | Eudragit® NE 30 D |
| 0,041 kg | Magnesiumstearat |
| 0,030 kg | Titandioxid |
| 0,018 kg | Polyethylenglykol 6000 |
| 0,021 kg | Polysorbat 80 |
| 4,237 kg | Wasser |
| 7,600 kg | Coatingsuspension |

Für den Sprühprozeß werden die folgenden Parameter eingestellt:

| | |
|---|---|
| Zulufttemperatur | 32 °C |
| Sprührate | 200 g/min |
| Sprühdüse | 12 Dreistoffdüsen, Durchmesser 0,8 mm |
| Sprühluftdruck | 1,5 bar |

Nach dem Coatingprozeß werden die Retardpellets bei 50 °C im Umluftrockenschrank für 48 Stunden getempert.

Die so erhaltenen Retardpellets weisen die folgende Zusammensetzung auf:

| | |
|---|---|
| 0,050 mg | Iloprost |
| 0,330 mg | β-Cyclodextrin |
| 56,628 mg | Lactose, 90 % < 0,1 mm |
| 6,334 mg | Avicel PH 101 |
| 1,374 mg | Eudragit® NE |
| 0,058 mg | Magnesiumstearat |
| 0,042 mg | Titandioxid |
| 0,025 mg | Polyethylenglykol 6000 |
| 0,030 mg | Polysorbat 80 |
| 0,129 mg | Siliciumdioxid |
| 65,000 mg | Retardpellets |

Die in vitro Freigabeuntersuchung einer abgeteilten Dosis im Dissolutiontest, Apparat 1, basket, USP in Phosphatpufferlösung pH 7,4 führte zu folgenden Werten:

| Zeitpunkt der Untersuchung | Freigabe nach 1 Stunde | Freigabe nach 2 Stunden | Freigabe nach 3 Stunden |
|---|---|---|---|
| Start (getempert: 50 °C/48 Stunden | 49,8 % | 74,6 % | 86,5 % |
| nach 4 Wochen Lagerung (getempert: 50 °C/48 Stunden) | 49,3 % | 75,1 % | 87,2 % |
| nach 9 Monaten Lagerung (getempert: 50 °C/48 Stunden) | 49,4 % | 71,4 % | 84,2 % |

Die Lagerung erfolgte bei Raumtemperatur. Das bei 50 °C über 48 h getemperte Produkt ist auch nach einer Lagerzeit von 9 Monaten stabil.

## Patentansprüche

1. Verfahren zur Herstellung von ummantelten, sphärischen Granulatkörner (Pellets), wobei das Verfahren die folgenden Schritte umfaßt:
a) in einem Wirbelbett oder Fließbett werden wirkstoffhaltige Rohpellets aus mindestens einem pharmazeutischem Wirkstoff und gegebenenfalls pharmazeutisch verträglichen Zusatzstoffen und / oder Trägem mit einer Polymerdisperson und Additiven besprüht, die eine Ethylcellulose und / oder ein Poly(meth)acrylsäureester als Polymer umfaßt,
β) eine Polymerdispersion wird mit einer Polymerdicke im Bereich von 1 bis 5 % (w/w) der Gesamtmasseaufgetragen,
wobei die Gesamtmasse der Zusammensetzung der ummantelten Pellets entspricht,
γ) die mit Polymer ummantelten Pellets werden bei einer Temperatur von 45 bis 65 °C getempert.
δ) die Temperung der mit dem Polymer ummantelten Pellets dauert mindestens 24 Stunden, und
ε) der pharmazeutische Wirkstoff ein Clathrat eines Prostan-Derivates ist.

2. Verfahren nach Anspruch 1, bei dem die Pellets in einem Gleichstromverfahren besprüht werden.

3. Verfahren nach Anspruch 2, bei dem aus mindestens 1 oder 2 Düsen die Polymerdispersion und gegebenenfalls zugesetzte Additive in das Wirbelbett oder Fließbett gesprüht wird.

4. Verfahren nach einem der Anspruche 1 bis 3, bei dem die ummantelten Pellets anschließend in eine Retardkapsel oder Tablette überführt werden.

5. Verfahren nach einem der vorherigen Ansprüche , wobei das Clathrat ein Cyclodextrin-Clathrat eines Prostan-Derivates ist.

6. Verfahren nach Anspruch 5 wobei das Prostan-Derivat 5-(E)-(1S,5S,6R)-7-Hydroxy-6[(E)-(3S,4RS)-3-hydroxy-4-methyl-1-octen-6-inyl]bicyclo [3.3.0]octen-3-ylidenpentansäure ist.

7. Verfahren nach einem der vorherigen Ansprüche, wobei die pharmazeutischen Wirkstoffe als niedrig dosierte Wirkstoffe vorliegen.

8. Verfahren nach einem der vorherigen Ansprüche, wobei das Polymer eine Dicke von 1,5 bis 4 % (w/w) der Gesamtmasse aufweist.

9. Verfahren nach einem der vorherigen Ansprüche, wobei beim Tempem eine Temperatur 45 bis 62 °C vorherrscht.

10. Verfahren nach einem der vorherigen Ansprüche, wobei das Tempem mindestens 48 h ± 5 Stunden dauert.

## Claims

1. Process for producing encased spherical granular grains (pellets) which comprises the steps of:
α) spraying active-ingredient-containing crude pellets which are composed of at least one active pharmaceutical ingredient and optionally pharmaceutically tolerable addition materials and/or carriers with a polymer dispersion and additives which comprises an ethyl cellulose and/or a poly(meth)acrylic ester as polymer, in a fluidized bed or moving bed,
β) applying a polymer dispersion to a polymer thickness in the range from 1% to 5% (w/w) of the total mass, the total mass corresponding to the composition of the encased pellets,
γ) heat treating the polymer-encased pellets at a temperature in the range from 45 to 65°C,
δ) the heat treatment of the polymer-encased pellets has a duration of not less than 24 hours, and
ε) the active pharmaceutical ingredient is a clathrate of a prostane derivative.

2. Process according to Claim 1 wherein the pellets are sprayed in a cocurrent process.

3. Process according to Claim 2 wherein the polymer dispersion and optionally added additives is sprayed into the fluidized bed or moving bed from at least one or two nozzles.

4. Process according to any one of Claims 1 to 3 wherein the encased pellets are subsequently transferred into a timed-release capsule or tablet.

5. Process according to any one of the preceding claims, wherein the clathrate is a cyclodextrin clathrate of a prostane derivative.

6. Process according to Claim 5 wherein the prostane derivative is 5-(E)-(1S,5S,6R)-7-hydroxy-6[(E)-(3S,4RS)-3-hydroxy-4-methyl-1-octen-6-ynyl]bicyclo-[3.3.0]octen-3-jrlidene pentanoic acid.

7. Process according to any one of the preceding claims wherein the active pharmaceutical ingredients are present as low-dosed active ingredients.

8. Process according to any one of the preceding claims wherein the polymer has a thickness ranging from 1.5% to 4% (w/w) of the total mass.

9. Process according to any one of the preceding claims wherein the heat treatment is carried out at a temperature in the range from 45 to 62°C.

10. Process according to any one of the preceding claims wherein the heat treatment has a duration of not less than 48 h ± 5 hours.

## Revendications

1. Procédé pour la préparation de granules sphériques, enrobés (pellets), le procédé comprenant les étapes suivantes :
α) les pellets bruts contenant une ou des substances actives, constitués par au moins une substance active pharmaceutique et le cas échéant des additifs et/ou des supports pharmaceutiquement acceptables sont aspergés dans un lit tourbillonnant ou fluidisé par une dispersion de polymère et d'additifs, qui comprend de l'éthylcellulose et/ou un poly(ester de l'acide (méth)acrylique) comme polymère,
β) on applique une dispersion de polymère à une épaisseur de polymère dans la plage de 1 à 5% (P/P) de la masse totale, la masse totale correspondant à la composition des pellets enrobés,
γ) les pellets enrobés de polymère sont traités thermiquement à une température de 45 à 65°C,
δ) le traitement thermique des pellets enrobés par le polymère dure au moins 24 heures et
ε) la substance active pharmaceutique est un clathrate d'un dérivé de prostane.

2. Procédé selon la revendication 1, dans lequel les pellets sont aspergés dans un procédé à courant parallèle.

3. Procédé selon la revendication 2, dans lequel la dispersion de polymère et les additifs le cas échéant ajoutés sont pulvérisés à partir d'au moins 1 ou 2 pulvérisateurs dans le lit tourbillonnant ou fluidisé.

4. Procédé selon l'une quelconque des revendications 1 à 3, dans lequel les pellets enrobés sont ensuite transformés en une capsule de retard ou un comprimé.

5. Procédé selon l'une quelconque des revendications précédentes, le clathrate étant un clathrate de cyclodextrine d'un dérivé de prostane.

6. Procédé selon la revendication 5, le dérivé de prostane étant l'acide 5-(E)-(1S,5S,6R)-7-hydroxy-6[(E)-(3S,4RS)-3-hydroxy-4-méthyl-1-octén-6-inyl]bicyclo [3.3.0]octén-3-ylidènepentanoïque.

7. Procédé selon l'une quelconque des revendications précédentes, les substances actives pharmaceutiques se trouvant sous forme de substances actives faiblement dosées.

8. Procédé selon l'une quelconque des revendications précédentes, le polymère présentant une épaisseur de 1,5 à 4% (P/P) de la masse totale.

9. Procédé selon l'une quelconque des revendications précédentes, une température de 45 à 62°C régnant pendant le traitement thermique.

10. Procédé selon l'une quelconque des revendications précédentes, le traitement thermique durant au moins 48 h ± 5 heures.
